# EUROPEAN PATENT SPECIFICATION

(11) **EP 1 583 964 B1**
(45) Date of publication and mention of the grant of the patent: **07.03.2007**
(21) Application number: 04701374.3
(22) Date of filing: 12.01.2004
(51) Int. Cl.: G01N 33/487

(54) **SENSOR DISPENSING DEVICE**
SENSORABGABEEINRICHTUNG
DISPOSITIF DE DISTRIBUTION DE DETECTEURS

(30) Priority: 14.01.2003 GB 0300765
(43) Date of publication of application: 12.10.2005
(73) Proprietor: HYPOGUARD LIMITED, Woodbridge Suffolk IP12 1PE (GB)
(72) Inventor: BRICKWOOD, David, Acton, London W3 7RT (GB); MAISEY, Graeme, Chessington, Surrey KT19 1JR (GB); MAY, Stuart, Richard, Kingston Upon Thames, Surrey KT2 6SB (GB)
(74) Representative: Gemmell, Peter Alan
(86) International application number: PCT/GB2004/000072
(87) International publication number: WO 2004/063747

(56) References cited:
- WO-A-94/10558
- US-A1- 2002 057 993
- PATENT ABSTRACTS OF JAPAN vol. 0112, no. 08 (P-593), 7 July 1987 (1987-07-07) & JP 62 030962 A (FUJI PHOTO FILM CO LTD), 9 February 1987 (1987-02-09)

## Description

### BACKGROUND OF THE INVENTION

### 1. Field of the Invention

The present invention relates to a device for dispensing sensors for measuring the concentration of an analyte in a fluid sample (notably glucose in whole blood), and to a cartridge containing sensors for use in the device. The invention also provides a meter incorporating the dispensing device.

### 2. Description of the Prior Art

Diabetics regularly need to test samples of their blood to determine the levels of blood glucose. In one known type of test system, disposable sensors are used to test the blood. The sensors typically take the form of test strips which are provided with a reagent material that will react with blood glucose to produce an electrical signal. Conductive tracks on the test strip relay the electrical signal to a meter which displays the result. After a sample of blood has been applied to the test strip and the measurement has been taken, the test strip is disposed of. Examples of test devices with test strip dispensers are described in US Patent No. 5,660,791, and European Patent Application Numbers 0 732 590, 0 738 666, and 0 811 843.

A problem with test strips is that they have only a limited shelf life, and exposure of test strips to the atmosphere further reduces the shelf life.

In DE 196 39 226 A1 it is proposed to provide a test device with a cartridge that may have a plurality of chambers containing test strips, each of which chambers may be individually sealed to preserve the shelf life of the strips therein. A user removes the seal for each chamber when required, and a timing circuit may be activated either by the user or when the cartridge is pushed into the device. After a set time period has elapsed, an alarm or other indication reminds the user that the time period for using the strips has elapsed. WO 02/08753 describes a blood glucose meter which has test strips arranged in a plurality of stacks in a magazine. Each stack is individually sealed, and the stack's seal is broken automatically when the magazine moves to a location where a test member can be dispensed by means of a suitable pusher.

It has been proposed in WO 94/10558 to provide a stack of disposable sensors in a cylindrical housing, the stack being urged towards a test station to form a liquid-proof seal. In WO 02/18940 there is disclosed a blood glucose test meter in which a stack of test strips in a replaceable cartridge are sealed against a rotatable transport member which is adapted to receive a single test strip and rotatably transport the test strip while maintaining a seal around the cartridge.

A problem with such systems is that the sealing means may wear with repeated use and the quality of the seal may consequently be reduced.

US 5,759,010 discloses a cartridge for dispensing slide test elements of the kind which have an opening for liquid access. The cartridge is provided with an internal cover plate which is biased to make a sealing contact with the opening so as to protect the inside of the opening from atmospheric moisture before the slide is dispensed. Such an arrangement is less desirable for test members in which the reagents are not located in an opening of a moisture-impermeable slide member because it is difficult to make a reliable seal around the reagents. Friction between the reagent layer and the plate may also tend to abrade the reagent layer.

It is an object of the present invention to provide an improved test device. It is a further object of the invention to provide an improved dispenser for sensors for use in measuring analyte concentration in an applied fluid.

### SUMMARY OF THE INVENTION

According to the present invention there is provided a cartridge for a sensor dispensing device for dispensing sensors for testing of analyte concentration in a fluid to be applied thereto,
the cartridge having an outer casing and a plurality of sensors arranged one upon another in a stack therein;
the cartridge having a first dispensing end and a second opposing end spaced a fixed distance apart, and the cartridge including a first aperture for the ejection of a sensor closest to the first end and a second aperture opposed to the first aperture, for access by a pushing member;
wherein the first aperture and the second aperture are each provided with compliant sealing means which are carried by the cartridge and which are at least partly disposed outside the outer casing, the sealing means having first and second sealing surfaces which are capable of co-operating to releasably form a substantially moisture-tight seal when acted upon by suitable clamping forces; wherein
each of the sealing means comprises a tube of a natural or synthetic rubber material. Suitable materials include styrene-ethylenebutylene-styrene (SEBS), for example Thermoflex^{™}, ethylene-propylene-diene monomer (EPDM) terpolymer, optionally alloyed with other materials such as polypropylene. Preferred materials are thermoplastic elastomers, for example Santoprene^{™}, a nitrile rubber mixed with polypropylene, or thermoplastic polyurethane elastomers, for example Pellethane^{™}. A preferred material is a mixture of Thermoflex^{™} 45A with Nourymix^{™} SP E60 antistatic/slip agent (from Akzo Nobel Chemicals). Nourymix^{™} SP E60 comprises 80% of a rapeseed oil-based erucamide (13-docosenamide) on a polypropylene carrier. The additive helps to prevent sticking of the tubular elastomer after being clamped for a length of time. The concentration of Nourymix^{™} is preferably in the range 0.2 to 5%, notably about 3%. Each tube may be disposed through its associated aperture and be a close fit for this aperture. Thus, when the tubes are suitably clamped or nipped the inside of the cartridge is sealed from moisture.

### BRIEF DESCRIPTION OF THE DRAWINGS

The invention will now be further described, by way of example only, with reference to the following drawings in which:
Figure 1 is a sectional view from underneath a blood glucose meter according to a first embodiment of the invention;
Figure 2 is a simplified cutaway view from the right side of the meter of Figure 1;
Figure 3 is a top view of the blood glucose meter of Figures 1 and 2;
Figure 4 is an end elevation view of a cartridge for the meter of Figures 1 to 3 in accordance with an embodiment of a further aspect of the invention;
Figure 5 is sectional view along the lines I-I of Figure 4;
Figures 6-8 are part-sectional views through the meter of Figure 3 from above the meter, from the handle end of the meter, and from below the meter respectively;
Figure 9 is a perspective view of an embodiment of a rubber sealing member for use in the invention;
Figures 10 to 14 are simplified perspective views of parts of a blood glucose meter in accordance with another embodiment of the invention;
Figures 15 and 16 are perspective views showing details of the latch spring mechanism of an embodiment of the invention;
Figures 17 and 18 illustrate alternative drive mechanisms in accordance with still further embodiments of the invention;
Figure 19 is an exploded view of one embodiment of a cartridge inner assembly for the cartridge of Figure 5; and
Figures 20 and 21 illustrate stages in the assembly of an alternative embodiment of a cartridge inner assembly for the cartridge of Figure 5.

### DETAILED DESCRIPTION

In the embodiments illustrated in the drawings, parts which perform the same function will be denoted by the same numbers.

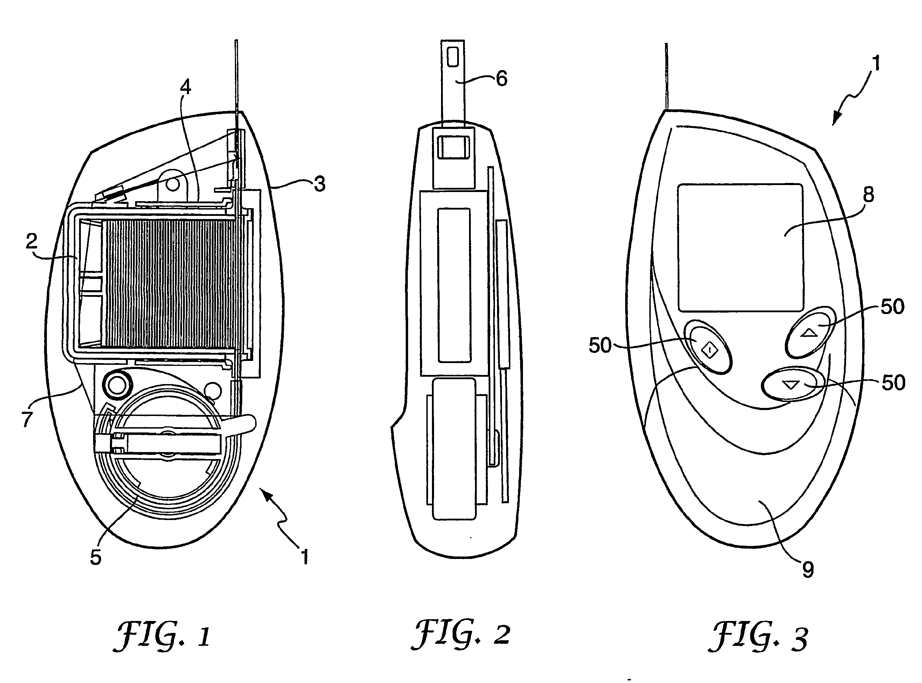

The blood glucose meter 1 shown in Figures 1 to 3 comprises an outer casing 3 which houses a cartridge 2 and a delivery mechanism 5 for dispensing test strips 6 from the cartridge 2. The casing 3 also houses a moveable clamp 4 for sealing the inside of the cartridge 2 from atmospheric moisture, as will be described in more detail below. The external features of the meter 1 comprise control buttons 50 for controlling the operation of the meter, an LCD 8 for displaying user instructions, results and other data, and an external handle 9 for actuating the delivery mechanism. A control PCB 7 is operably connected to the LCD 8 and buttons 50. The meter 1 of Figures 1-3 is shown with a test strip 6 in a dispensed position ready to receive a drop of blood.

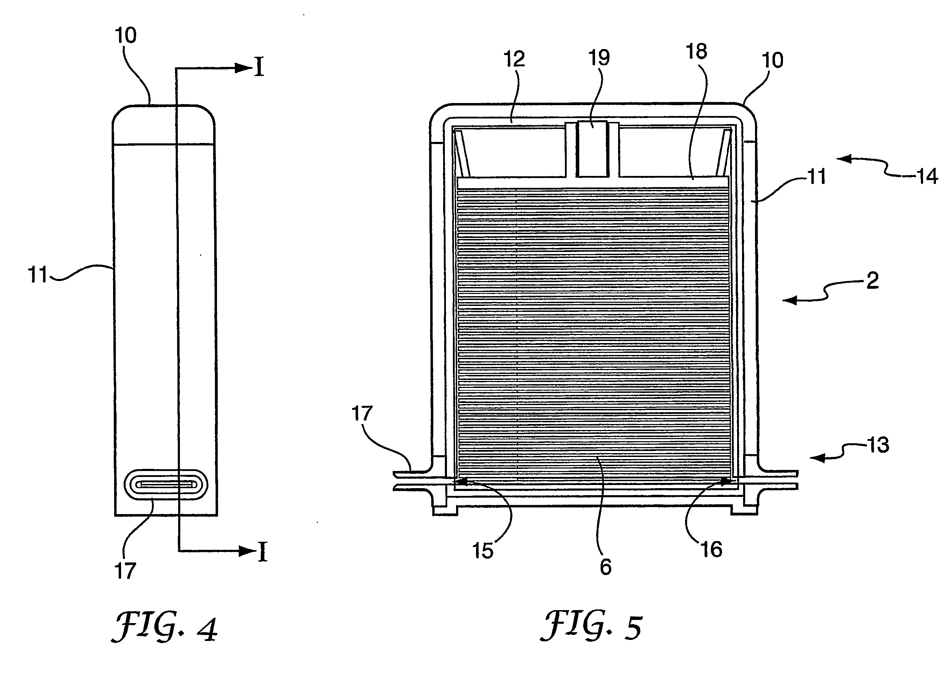

Referring now to Figures 4 and 5, the cartridge 2 has an inner assembly 12 and an outer casing 11, in this example formed from polypropylene, sealingly covered by a cap 10. The cartridge has a first, dispensing, end 13 and an opposing end 14 which in this embodiment includes the cap 10. The cap 10 may be welded to the remainder of the outer casing, for example by ultrasonic welding, to form a fluid-tight bond. Instead of a cap, the outer casing may be closed by foil, for example of aluminium, or other suitable sealing member.

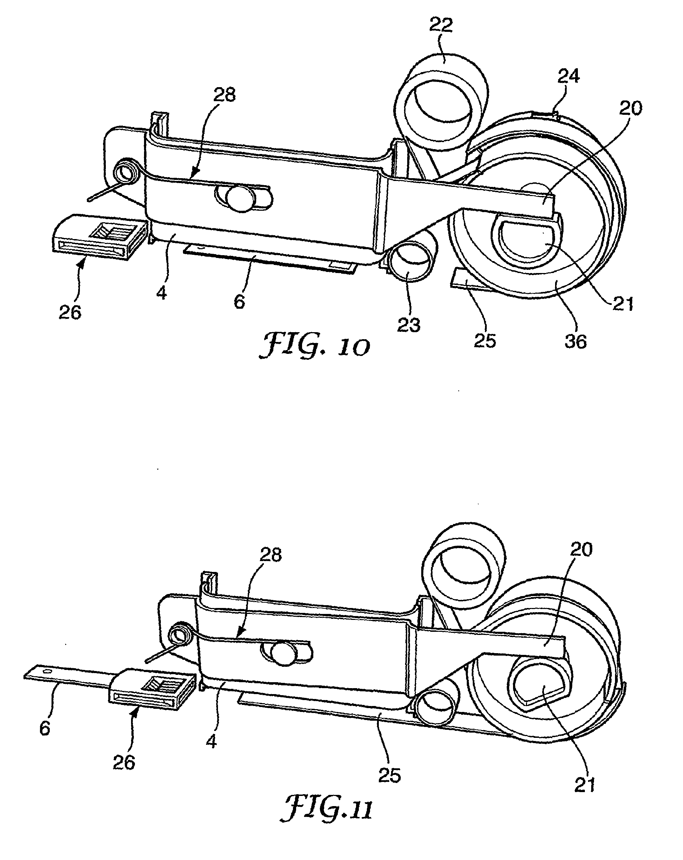

A stack of test strips 6 is housed in the cartridge inner assembly 12, and the strips 6 are urged towards the dispensing end by a constant tension spring 19 which acts on a follower 18. At the dispensing end 13 there are opposed first 15 and second 16 apertures, each of which is provided in this embodiment with a co-moulded tubular rubber sealing member 17, as best shown in Figure 9. The sealing members 17 comprise a first sealing lip 17a and a second sealing lip 17b, each sealing lip providing a sealing surface. In this embodiment, the sealing surfaces are provided as part of a single tubular member 17, but they could alternatively be separately provided. When the sealing members 17 are open they permit a pusher to be inserted through one aperture to push a test strip 6 through the other aperture. When the sealing members 17 are clamped shut, the inside of the outer casing 11 is substantially sealed off from atmospheric moisture. The cartridge 2 will be kept in a moisture-tight container (not shown) until immediately prior to its insertion into the meter 1.

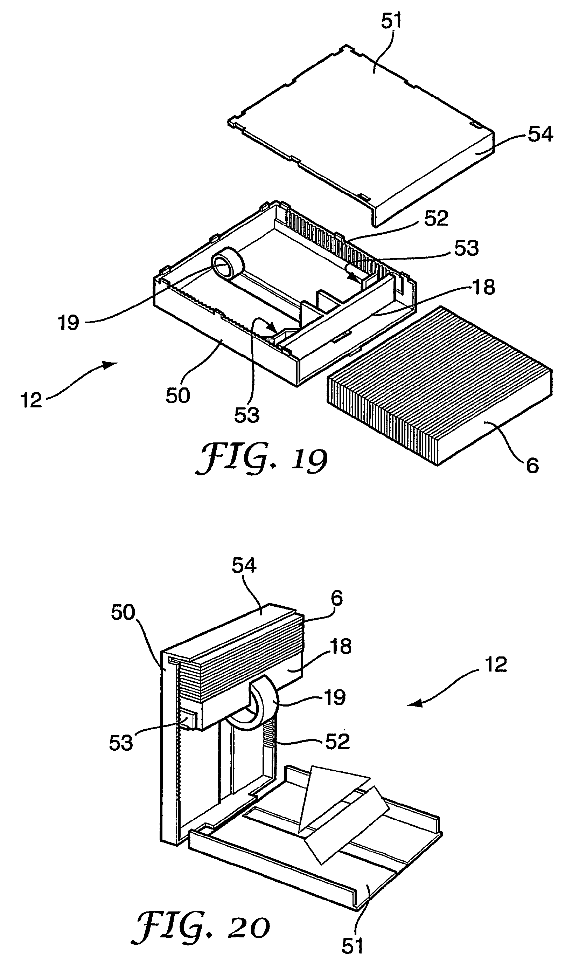

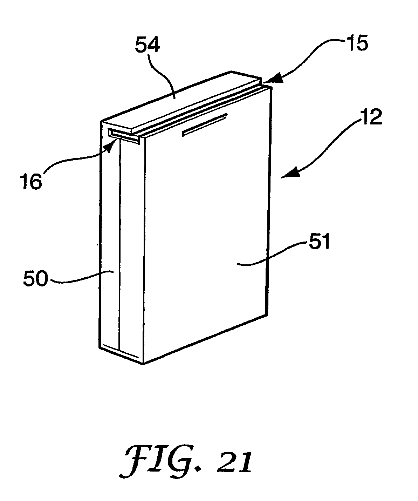

One way of manufacturing the cartridge inner assembly 12 is illustrated in the exploded diagram shown in Figure 19. The walls of the cartridge inner assembly 12 are formed from a base member 50 and a closure member 51. Two opposed upstanding walls of the base member 50 are provided with a series of ridges 52 in which fit arms 53 of the follower 18. The ridges 52 and arms 53 are profiled to permit movement of the follower 18 in one direction only, towards the stack of test strips 6. During assembly, the follower 18 is located near to the spring 19 to permit the stack of strips 6 to fit in the base member 50. The closure member 51 is snap-fitted on the base member 50 to form the cartridge inner assembly 12. A lip 54 on the closure member 51 provides a stop member which limits outward travel of the strips 6. There is a sufficient gap between the lip 54 and the adjacent walls of the base member 50 (which define opposed openings of the housing) to permit a single strip 6 to slide out axially. An alternative design of cartridge inner assembly 12 is shown in Figures 20 and 21. Here, the stop member 54 is provided on the base member 50.

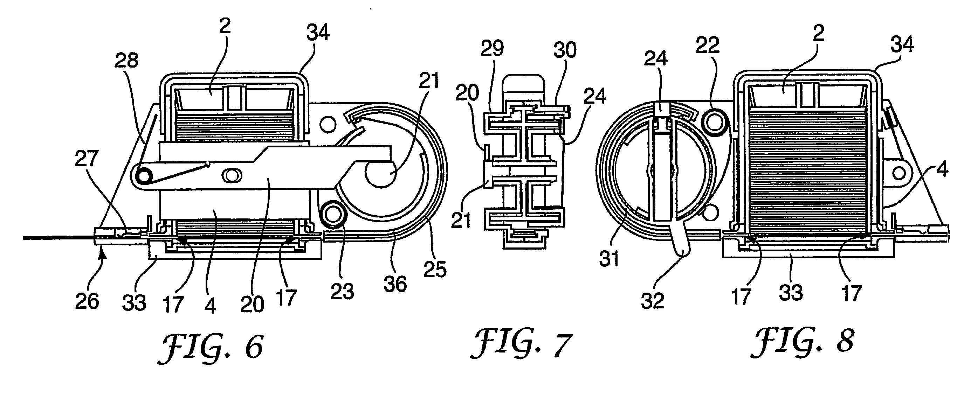

Referring now to Figures 6-8, the working parts of the meter 1 are mounted on a chassis comprising a first chassis member 29 and a second chassis member 30. The cartridge 2 is received in a cartridge-receiving portion 34 in the meter casing 3. A lid 33, is closed over the dispensing end of the cartridge 2 and provides a shoulder on which the tubular sealing members 17 rest. The clamp 4 is urged towards the lid 33 by a clamping spring 28. The clamp 4 is operatively connected to by a clamp arm 20 to a rotatable arm lift cam 21. In the rest position shown in Figure 6, the bottom edges of the clamp 4 exert a clamping force on the tubular sealing members 17 so as to clamp the sealing members 17 between the clamp 4 and inner surfaces of the lid 33, thereby providing a substantially fluid-tight seal to protect the inside of the cartridge 2 from the external atmosphere. The delivery mechanism comprises a pusher drum 36 on which is wound an axially elongate pusher 25, and a drive drum 31 which has a drive handle 32 operatively connected to the external handle 9 of the meter. A latch spring 24 is provided on the drive drum 31 for releasably engaging the drive drum 31 with the pusher drum 36. It will be understood that the drive drum and the pusher drum need not be hollow, and could comprise solid cylinders, wheels, discs or the like. It is preferred that the drums are substantially circular in cross section, but other shapes such as an oval could also be used.

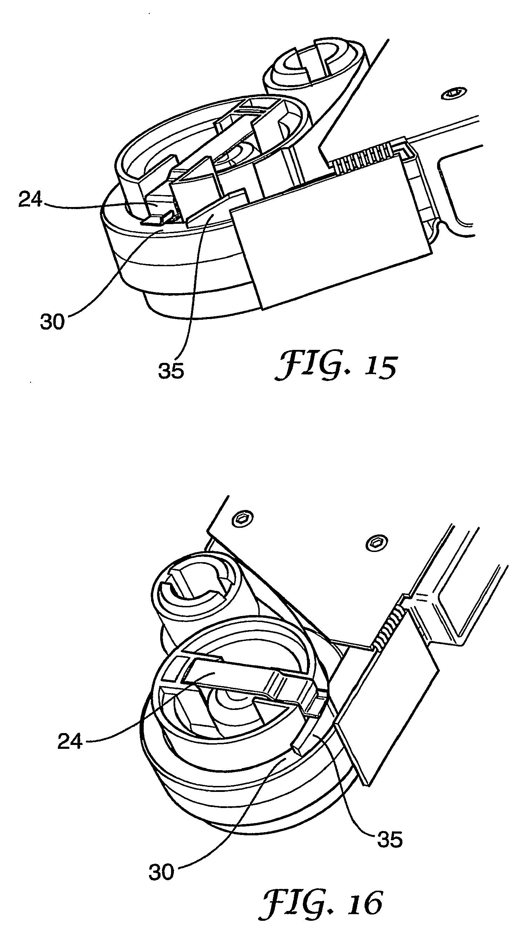

In the rest position the latch spring 24 is disengaged by a ramp 35 which is part of the second chassis member 30, as best shown in Figure 16. As the user operates the external handle 9, the handle 32 of the drive drum turns the drive drum 31. The drive drum 31 is free to turn through a set angle with the latch spring 24 turning with it. A drive spring 22, which connects the drive drum 31 directly or indirectly to the chassis, is wound up. Nothing happens to the pusher 25 during the initial turning as it is held with a one-way ratchet feature. If the user releases the drive drum at this point the mechanism will return to the rest position without dispensing a test strip 6. At the "point of no return" the latch spring 24 drops into a slot on the pusher drum 36, effectively locking the two drums together. At this point the pusher 25 is in its rest position. When the user lets go the handle 9, the drive drum 31 and pusher drum 36 are forced to rotate by the wound-up drive spring 22. During this rotation three things happen:
the arm lifting cam 21 lifts the clamp arm 20 to open the tubular sealing members 17 (Figure 8);
the flexible pusher 25 forces a test strip 6 from the cartridge 2 to a dispensed position under meter contacts 27 within a contact block 26; and
a return spring 23, which connects the pusher drum 36 directly or indirectly to the chassis, is wound up.
At the end of the rotation of the drive drum 31, the latch spring 24 is lifted out of the slot in the pusher drum 36 by the ramp 35. The relative positions of the latch spring 24 before and after engagement with the ramp 35 are illustrated in Figures 15 and 16 respectively.

When the pusher drum 36 is released from the drive drum 31 it returns to its rest position by the action of the return spring 23. At the end of this rotation the arm lift cam 21 permits the clamp arm 20 to drop and reestablish a clamping force across the sealing members 17.

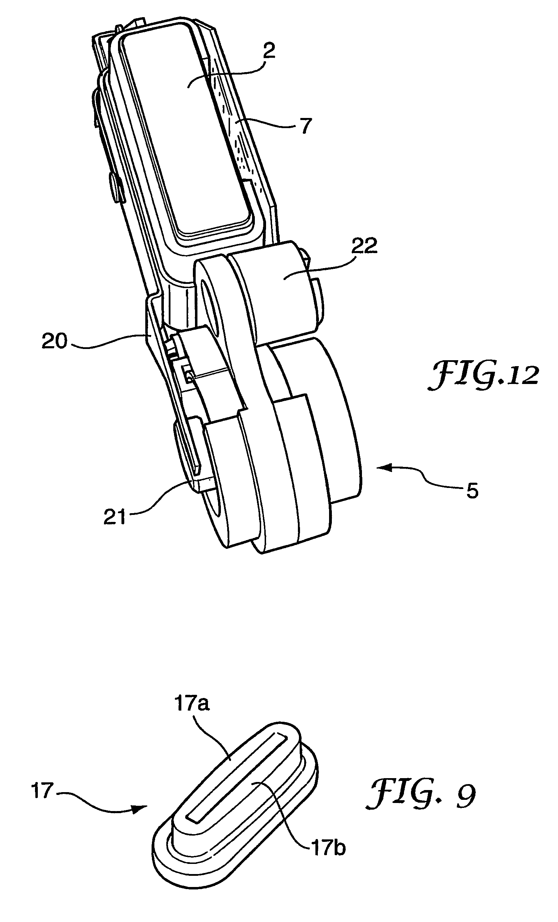

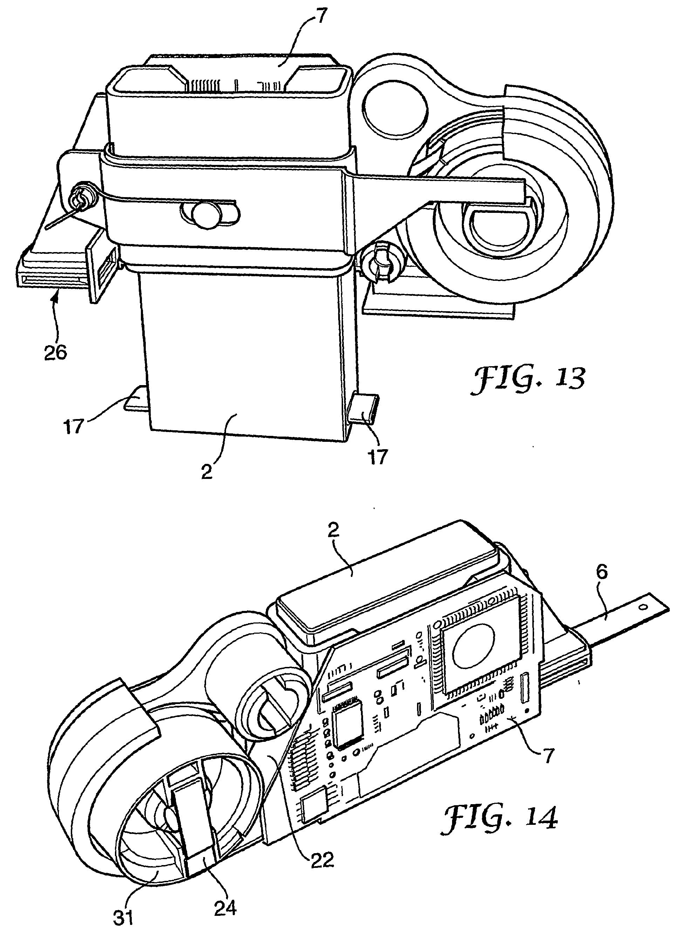

Referring now to Figure 10, a simplified view of an alternative embodiment of the invention illustrates the location of the test strip 6 prior to being dispensed. The pusher 25 is in an undeployed state. In Figure 11 the arm lift cam 21 has lifted the clamp arm 20 and the clamp 4. The pusher 25 has been deployed so as to push the test strip 6 to the dispensed position where its electrodes are in contact with meter contacts in the contact block 26. The pusher 25 is no longer fully deployed and is in the process of being retracted onto the pusher drum. Simplified figures 12-14 illustrate parts of the meter with the cartridge 2 at different stages of insertion.

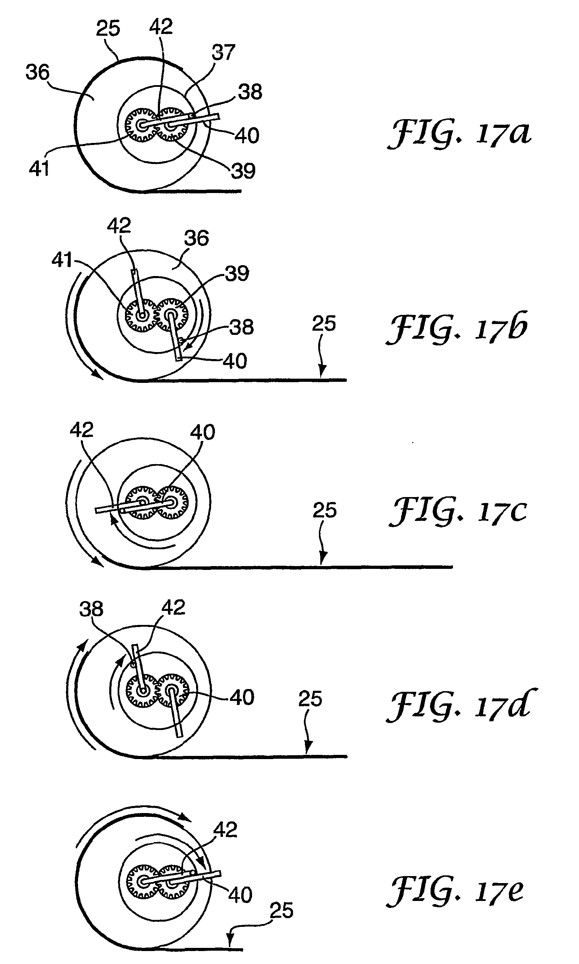

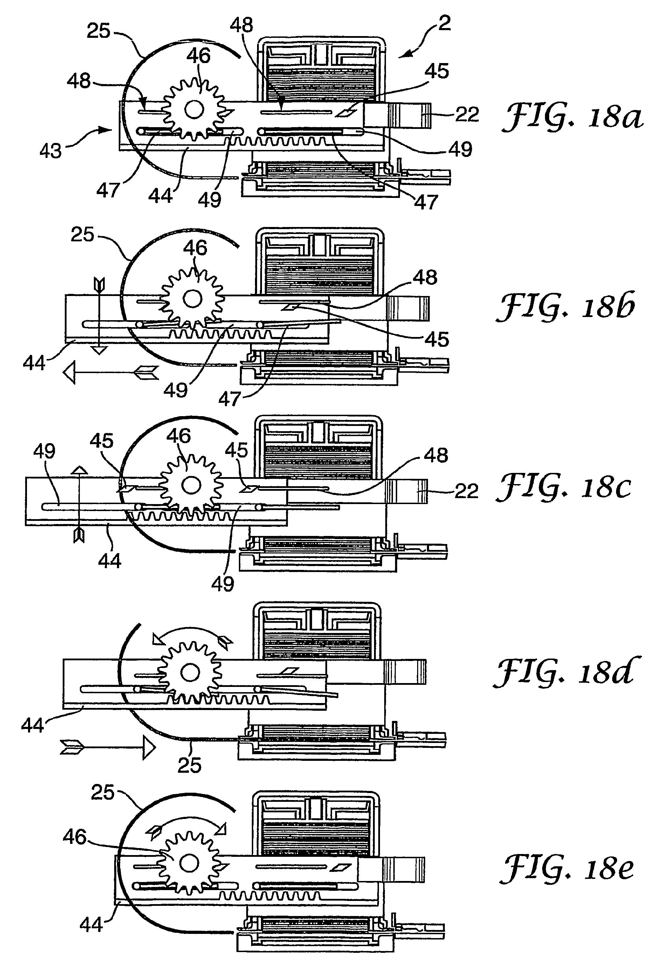

The delivery system of the meter is mechanically robust and uses simple moulded components. The mechanism permits a more symmetrical product to be manufactured because the delivery mechanism 5 sits behind the cartridge 2, as best shown in Figure 12. The mechanism may be operated by either rotary or linear user activation. Alternative mechanical systems to control clamping of the sealing members and co-ordinated deployment of the pusher are described below with reference to Figures 17 and 18.

The delivery mechanism illustrated in Figure 17 comprises drive disc 37 on which is mounted a pin 38. The drive disc 37 is connected to the arm lift cam 21 (not shown). A first transfer pinion 39 and a second transfer pinion 41 are provided with, respectively, first 40 and second 42 transfer blades. The transfer pinions 39, 41 are rotatably mounted in relation to the drive disc 37 and their teeth are interengaged. The second transfer pinion 41 is directly linked to the pusher drum 36. The mechanism works as follows.
1. With the mechanism arranged as in Figure 17a, the user winds the activating handle through 120°.
2. This action winds up the drive spring which is connected to the drive disc 37.
3. Once the user passes the "point of no return" the drive disc 37 is free to begin its 360° rotation using the energy stored in the drive spring (Figure 17b).
4. This rotation forces the pin 38 on the drive disc 37 to push on the first transfer blade 40 which pushes the first transfer pinion 39 through 180°.
5. Pushing the first transfer pinion 39 clockwise makes the second transfer pinion 41 rotate counter-clockwise as they are directly meshed together. Turning of the second transfer pinion 41 turns the pusher drum 36 and the flexible pusher 25 is deployed (Figure 17c) and forces a test strip to the deployed position.
6. After 180° of rotation the pin 38 slips off the first transfer blade 40 and begins to act on the second transfer pinion 41 via the second transfer blade 42. This reverses the direction of the pusher drum 36, retracting the pusher 25 (Figure 17d).
7. At the end of the 360° rotation of the drive disc 37, the pin 38 slips off the second transfer blade 42 returning the mechanism to the rest state and completing the mechanical movement (Figure 17e).

This system can readily be driven by an electric motor because the drive disc is driven in only one direction. Alternatively, it may be actuated by either linear or rotary user activation. It uses simple moulded components, some of which are repeated. This mechanism can also be located behind the cartridge, permitting a symmetrical product design. Because the mechanism self-reverses, no opposing spring force is required.

Referring now to Figure 18, a further alternative delivery mechanism is illustrated, which uses a rack and pinion arrangement. The mechanism works as follows:
1. The user pulls back the external handle; this pulls back a slide 43 from the rest position shown in Figure 18a and winds up the return spring. The slide 43 is provided with a rack 44 for engagement with a pinion 46.
2. As the slide 43 travels backwards, diamond-shaped lugs 45 on the slide engage with fixed ribs 48 on the chassis. The lugs 45 deflect below the ribs 48 (Figure 18b) causing the rack 44 to miss the pinion 46. At the same time, sprung pins 47 slide in grooves 49.
3. At the position shown in Figure 18c, the slide 43 has reached the end of its backwards travel and the lugs 45 disengage from the ribs 48, allowing the slide 43 to be pushed up by the sprung pins 47. This movement causes engagement of the rack 44 and the pinion 46.
4. The tensator spring 22 now pulls the rack (Figure 18d), driving the pinion 46 which in turn drives the pusher drum (not shown) and deploys the pusher 25 to eject a test strip. The diamond-shaped lugs 45 now sit on the top side of the fixed ribs 48.
5. The rack 44 goes beyond the pinion 46 and a return spring withdraws the pusher 25. The lugs 45 drop off the ribs 48, leaving the mechanism in the rest position (Figure 18e).

The mechanism is mechanically simple and uses simple moulded components.

Although the invention has been described with reference to a sensor dispensing device or test device for measuring blood glucose concentration, it is to be understood that the invention is not limited to this application. The invention may be used in the determination of any analyte in a fluid, biological or otherwise, by the use of suitable reagents in the test strip. Such reagents are well known to those skilled in the art.

## Claims

1. A cartridge (2) for use in a sensor dispensing device, having an outer casing (11) and a plurality of sensors (6) arranged one upon another in a stack therein, each sensor (6) being for testing of analyte concentration in a fluid to be applied thereto;
the cartridge having a first dispensing end (13) and a second opposing end spaced a fixed distance apart, and the cartridge including a first aperture (15) for the ejection of a sensor closest to the first end (13) and a second aperture (16) opposed to the first aperture, for access by a pushing member;
wherein the first aperture (15) and the second aperture (16) are each provided with compliant sealing means (17) which are at least partly disposed outside the outer casing, each of the sealing means (17) comprising a tube of natural or synthetic rubber having first and second sealing surfaces (17a, 17b) which are capable of co-operating to releasably form a substantially moisture-tight seal when acted upon by suitable clamping forces.

2. A sensor dispensing device for dispensing sensors for testing of analyte concentration in a fluid to be applied thereto, the device comprising: a cartridge according to claim 1,
a housing for receiving the cartridge;
for each of the said compliant sealing means, a pair of clamping members for releasably clamping the sealing means to form a substantially moisture-tight seal; and
a pushing member for reversible insertion through the second aperture when the sealing means are not clamped, for pushing the sensor closest to the first end through the first aperture to a dispensed position.

3. A device according to claim 2 wherein each of the sealing means is formed from a thermoplastic elastomeric material.

4. A device according to claim 2 or claim 3, wherein the sealing means are co-moulded with the outer casing of the cartridge.

5. A device according to any of claims 2-4, wherein the outer casing of the cartridge contains an inner assembly comprising an inner casing in which is located the stack of sensors and which has opposed apertures in register with the corresponding apertures in the outer casing, for permitting entry of the pushing member and exit of a sensor.

6. A device according to claim 5, further comprising spring means within the inner casing which urge the stack of sensors towards the dispensing end, and further comprising ratchet means within the inner casing which prevent or inhibit movement of the stack of sensors towards the opposing end.

7. A device according to claim 5 or claim 6 wherein the cartridge inner casing is formed from a desiccant plastics material.

8. A device according to any of claims 2-7, further including a chassis connected to the housing and a delivery mechanism for deploying the pushing member from a rest position outside the cartridge to a deployed position in which it will push a sensor from the cartridge to the dispensed position.

9. A device according to claim 8, wherein the delivery mechanism further includes a pusher drum rotatably mounted on the chassis, and wherein the pushing member is flexible and is at least partly wound around the pusher drum when the sealing members are clamped.

10. A device according to claim 8 or claim 9, wherein actuation of the delivery mechanism to deploy the pushing member will cause unclamping of the sealing means before the pushing member enters the second aperture and wherein withdrawal of the pushing member from the deployed position to the rest position will cause clamping of the sealing means after the pushing member withdraws from the second aperture.

11. A device according to claim 10, wherein the delivery mechanism further includes a cam which is connected to the drive drum and rotatable therewith; wherein initial rotation of the cam will cause the clamping members to unclamp the sealing means, and further rotation of the cam will cause the clamping members to clamp the sealing means.

12. A device according to claim 11, wherein the delivery mechanism includes a drive drum and a drive spring connecting the drive drum and the chassis; the drive drum being rotatably mounted in relation to the pusher drum, and the drive drum being turnable by a user from an initial position so as to wind up the drive spring, and wherein the delivery mechanism further comprises latch means for releasably connecting the drive drum and the pusher drum when the drive drum has turned from its initial position to a pre-determined engagement position so that returning of the drive drum from its engagement position to its initial position caused by unwinding of the drive spring will rotate the pusher drum.

13. A device according to claim 12, further including a return spring connecting the pusher drum and the chassis, wherein rotation of the pusher drum from an initial position by the drive drum will wind up the return spring so that when the pusher drum becomes disengaged from the drive drum the return spring will return the pusher drum to its initial position.

14. A device according to claim 9, wherein the delivery mechanism further comprises a first pinion wheel and a second pinion wheel and a drive member which is rotationally mounted in relation to the chassis; wherein the said pinion wheels are meshed together and one of the pinion wheels is fixed to the pusher drum so that they will rotate together; the arrangement being such that during a full rotation of the drive member it will engage with and turn the first pinion wheel for one part of its travel and will engage with and turn the second pinion wheel for a second part of its travel, whereby the pusher drum will be initially driven so as to cause the pushing member to extend from the rest position to the deployed position and will then be reversed so that the pushing member will be returned to the rest position.

15. A device according to claim 9, wherein the delivery mechanism further comprises a pinion wheel which is fixed to the pusher drum so that they will rotate together, and a rack mounted for reciprocal translation in relation to the chassis; the arrangement being such that during translation of the rack in a first direction it will not engage with the pinion and during translation of the rack in a second opposite direction it will engage with and turn the pinion so as to turn the pusher drum; the device further including a return spring connecting the pusher drum and the chassis, wherein rotation of the pusher drum from an initial position will wind up the return spring so that when the pinion becomes disengaged from the rack the return spring will return the pusher drum to its initial position.

16. A device according to any of claims 2-15, wherein the sensors comprise biosensors having reagent means thereon for producing an electrical signal in response to the concentration of analyte in an applied fluid, and electrode tracks in contact with the reagent means, and wherein the device further comprises electrical contacts mounted in relation to the housing for engaging with the said electrode tracks at the said dispensed position, and a meter connected to the contacts having electronics means for producing a signal output which is dependent on the signal from a sensor when the sensor is engaged with the contacts.

17. A device for measuring analyte concentration in a fluid, comprising a sensor dispensing device according to claim 2, with each biosensor having reagent means thereon for producing an electrical signal in response to the concentration of analyte in an applied fluid, and electrode tracks in contact with the reagent means;
the device further comprising:
electrical contacts mounted in relation to the housing for engaging with the said electrode tracks at the said dispensed position, and a meter connected to the contacts having electronics means for producing a signal output which is dependent on the signal from a sensor when the sensor is engaged with the contacts.

18. A cartridge according to claim 1 wherein each of the sealing means is formed from a thermoplastic elastomeric material.

19. A cartridge according to any of claims 1 or 18 wherein the sealing means are co-moulded with the outer casing of the cartridge.

20. A cartridge according to any of claims 1, 18 or 19 wherein the outer casing of the cartridge contains an inner assembly comprising an inner casing in which is located the stack of sensors and which has opposed apertures in register with the corresponding apertures in the outer casing, for permitting entry of the pushing member and exit of a sensor.

21. A cartridge according to claim 20, further comprising spring means within the inner casing which urge the stack of sensors towards the dispensing end, and further comprising ratchet means within the inner casing which prevent or inhibit movement of the stack of sensors towards the opposing end.

22. A cartridge according to claim 19 or claim 20 wherein the cartridge inner casing is formed from a desiccant plastics material.

23. A cartridge according to claim 18, wherein the sealing member comprises an ethylene-propylene-diene terpolymer.

24. A cartridge according to claim 23, wherein the sealing member further comprises from 0.2 to 5% of an erucamide antistatic/slip agent.

## Patentansprüche

1. Kassette (2) zum Gebrauch in einer Sensorabgabeeinrichtung mit einem Außengehäuse (11) und einer Vielzahl von darin aufeinander in einem Stapel angeordneten Sensoren (6), wobei jeder Sensor (6) zum Testen der Analytkonzentration in einem darauf aufzutragenden Fluid dient,
wobei die Kassette ein erstes Abgabeende (13) und ein zweites, gegenüberliegendes Ende, das eine bestimmte Distanz davon beabstandet ist, besitzt und die Kassette eine erste Öffnung (15) zum Ausstoßen eines dem ersten Ende (13) am nächsten kommenden Sensors und eine der ersten Öffnung gegenüberliegend angeordnete zweite Öffnung (16), die über ein Druckglied zugänglich ist, enthält,
und wobei die erste Öffnung (15) und die zweite Öffnung (16) jeweils mit nachgiebigen Dichtmitteln (17) ausgestattet sind, die zumindest teilweise außerhalb des Außengehäuses angeordnet sind, wobei jedes der Dichtmittel (17) ein Rohr aus natürlichem oder synthetischem Gummi mit einer ersten und einer zweiten Dichtfläche (17a, 17b) besitzt, die kooperieren können, um eine im Wesentlichen feuchtigkeitsdichte, lösbare Dichtung bilden, wenn auf sie durch geeignete Klemmkräfte eingewirkt wird.

2. Sensorabgabevorrichtung zur Abgabe von Sensoren zum Testen einer Analytkonzentration in einem darauf aufzutragenden Fluid, wobei die Vorrichtung aufweist:
eine Kassette nach Anspruch 1,
ein Gehäuse zur Aufnahme der Kassette,
ein paar von Klemmgliedern für jedes der nachgiebigen Dichtmittel zum lösbaren Klemmen der Dichtmittel, um eine im Wesentlichen feuchtigkeitsdichte Dichtung zu bilden, und
ein Druckglied zum reversiblen Einführen durch die zweite Öffnung, wenn die Dichtmittel nicht geklemmt sind, um den dem ersten Ende benachbartesten Sensor durch die erste Öffnung in eine Abgabeposition zu drücken.

3. Vorrichtung von Anspruch 2, bei der jedes der Dichtmittel aus einem thermoplastischen elastomeren Material gebildet ist.

4. Vorrichtung nach Anspruch 2 oder Anspruch 3, bei der die Dichtmittel mit dem Außengehäuse der Kassette co-geformt sind.

5. Vorrichtung nach einem der Ansprüche 2 bis 4, bei der das Außengehäuse der Kassette eine innere Baugruppe enthält, die ein Innengehäuse aufweist, in dem der Stapel von Sensoren angeordnet ist und das in Ausrichtung mit den entsprechenden Öffnungen in dem Außengehäuse gegenüberliegende Öffnungen besitzt, um den Eintritt des Druckgliedes und den Austritt eines Sensors zu ermöglichen.

6. Vorrichtung nach Anspruch 5, die außerdem innerhalb des Innengehäuses Federmittel aufweist, welche den Stapel von Sensoren zum Abgabeende drücken, und die außerdem Sperrklinkenmittel innerhalb des Innengehäuses aufweist, welche die Bewegung des Stapels von Sensoren in Richtung des gegenüberliegenden Endes verhindern oder hindern.

7. Vorrichtung nach Anspruch 5 oder Anspruch 6, bei der das Innengehäuse der Kassette aus einem trocknenden Kunststoffmaterial gebildet ist.

8. Vorrichtung nach einem der Ansprüche 2 bis 7, die außerdem aufweist ein mit dem Gehäuse verbundenes Chassis und einen Liefermechanismus zum Ausfahren des Druckgliedes aus einer Ruheposition außerhalb der Kassette in eine ausgefahrene Position, in der es einen Sensor aus der Kassette zur Abgabeposition drückt.

9. Vorrichtung nach Anspruch 8, bei der der Liefermechanismus außerdem eine drehbar an dem Chassis befestigte Schiebetrommel aufweist und bei der das Druckglied flexibel ist und zumindest teilweise um die Schiebetrommel gewickelt ist, wenn die Dichtglieder geklemmt sind.

10. Vorrichtung nach Anspruch 8 oder Anspruch 9, bei der die Betätigung des Liefermechanismus zum Ausfahren des Druckgliedes ein Entklemmen der Dichtmittels bewirkt, bevor das Druckglied in die zweite Öffnung eintritt, und bei der das Zurückziehen des Druckgliedes aus der ausgefahrenen Position in die Ruheposition ein Klemmen der Dichtmittel bewirkt, nachdem das Druckglied aus der zweiten Öffnung herausgezogen ist.

11. Vorrichtung nach Anspruch 10, bei der der Liefermechanismus außerdem eine Nocke aufweist, die mit der Antriebstrommel sowie damit drehbar verbunden ist, und bei der die anfängliche Drehung der Nocke die Klemmglieder dazu veranlasst, die Dichtmittel zu entklemmen, wobei eine weitere Drehung der Nocke die Klemmglieder dazu veranlasst, die Dichtmittel zu klemmen.

12. Vorrichtung nach Anspruch 11, bei der der Liefermechanismus eine Antriebstrommel und eine Antriebsfeder, welche die Antriebstrommel und das Chassis verbindet, aufweist, wobei die Antriebstrommel drehbar bezüglich der Schiebetrommel montiert ist, und die Antriebstrommel durch einen Benutzer aus einer anfänglichen Position gedreht werden kann, um die Antriebsfeder aufzuwickeln, und bei der der Liefermechanismus außerdem Klinkenmittel zum lösbaren Verbinden der Antriebstrommel mit der Schiebetrommel besitzt, wenn sich die Antriebstrommel von ihrer anfänglichen Position in eine vorherbestimmte Eingriffsposition gedreht hat, so dass die durch das Abwickeln der Antriebsfeder verursachte Rückkehr der Antriebstrommel von ihrer Eingriffsposition in ihre anfängliche Position die Schiebetrommel dreht.

13. Vorrichtung nach Anspruch 12, die außerdem eine Rückholfeder aufweist, welche die Schiebetrommel und das Chassis verbindet, wobei die Drehung der Schiebetrommel aus einer anfänglichen Position durch die Antriebstrommel ein Aufwickeln der Rückholfeder bewirkt, so dass, wenn die Schiebetrommel außer Eingriff von der Antriebstrommel gelangt, die Rückholfeder die Schiebetrommel in ihre anfängliche Position zurückbringt.

14. Vorrichtung nach Anspruch 9, bei der der Liefermechanismus außerdem umfasst ein erstes Ritzelrad und ein zweites Ritzelrad sowie ein Antriebsglied, das bezüglich des Chassis drehbar befestigt ist, und bei der die Ritzelräder ineinander greifien und eines der Ritzelräder an der Schiebetrommel fixiert ist, so dass sie zusammen rotieren, wobei die Anordnung derart ist, dass das Antriebsglied bei einer vollen Drehung des Antriebsgliedes mit dem ersten Ritzelrad für einen Abschnitt seiner Bewegung in Eingriff kommt und es dreht und mit dem zweiten Ritzelrad für einen zweiten Abschnitt seiner Bewegung in Eingriff kommt und es dreht, wobei die Schiebetrommel am Anfang derart angetrieben wird, dass das Druckglied von der Ruheposition in die ausgefahrene Position ausgefahren wird und dann rückwärts bewegt wird, so dass das Druckglied in die Ruheposition zurückkehrt.

15. Vorrichtung nach Anspruch 9, bei der der Liefermechanismus außerdem ein Ritzelrad, das an der Schiebetrommel derart befestigt ist, dass sie sich zusammen drehen, und eine Zahnstange, die für eine reziproke Translation in Richtung des Chassis befestigt ist, aufweist, wobei die Anordnung derart ist, dass die Zahnstange bei der Translation der Zahnstange in eine erste Richtung mit dem Ritzel nicht in Eingriff kommt und bei der Translation der Zahnstange in eine zweite entgegengesetzte Richtung mit dem Ritzel in Eingriff kommt und dreht, so dass die Schiebetrommel gedreht wird, wobei die Vorrichtung außerdem eine Rückholfeder aufweist, welche die Schiebetrommel und das Chassis verbindet, wobei die Drehung der Schiebetrommel aus einer anfänglichen Position die Rückholfeder aufwickelt, so dass, wenn das Ritzel aus der Zahnstange außer Eingriff gelangt, die Rückholfeder die Schiebetrommel in ihre ursprüngliche Position zurück bringt.

16. Vorrichtung nach einem der Ansprüche 2 bis 15, bei der die Sensoren Biosensoren, die über darauf befindliche Reagenzmittel zur Herstellung eines elektrischen Signals in Ansprechen auf die Konzentration des Analyten in einem aufgetragenen Fluid verfügen, und Elektronenbahnen, die in Kontakt mit den Reagenzmitteln sind, umfassen, und bei der die Vorrichtung außerdem elektrische Kontakte, die bezüglich des Gehäuses derart montiert sind, dass sie mit den Elektrodenbahnen an der Abgabeposition in Eingriff gelangen, und einen Zähler, der mit den Kontakten mit den elektronischen Mitteln zur Herstellung eines Signaloutputs, das von dem Signal eines Sensors abhängig ist, wenn der Sensor mit den Kontakten im Eingriff ist, verbunden ist, aufweist.

17. Vorrichtung zur Messung der Analytkonzentration in einem Fluid, aufweisend eine Sensorabgabevorrichtung nach Anspruch 2, wobei die Vorrichtung weiterhin aufweist: elektrische Kontakte die bezüglich des Gehäuses derart montiert sind, dass sie mit den Elektrodenbahnen an der Abgabeposition in Eingriff gelangen, und einen Zähler, der mit den Kontakten mit elektronischen Mitteln zur Herstellung eines Signaloutputs, das abhängt von dem Signal von einem Sensor, wenn der Sensor mit den Kontakten in Eingriff ist, verbunden ist.

18. Kassette nach Anspruch 1, bei der jedes der Dichtmittel aus einem thermoplastischen elastomeren Material gefertigt ist.

19. Kassette nach einem der Ansprüche 1 oder 18, bei der die Dichtmittel zusammen mit dem Außengehäuse der Kassette co-geformt sind.

20. Kassette nach einem der Ansprüche 1, 18 oder 19, bei der das Außengehäuse der Kassette eine Innenanordnung enthält, die ein Innengehäuse umfasst, in der der Stapel von Sensoren platziert ist und die gegenüberliegende Öffnungen in Ausrichtung mit den entsprechenden Öffnungen in dem Außengehäuse besitzt, so dass der Eintritt des Druckgliedes und der Austritt eines Sensors ermöglicht wird.

21. Kassette nach Anspruch 20, die außerdem Federmittel innerhalb des Innengehäuses umfasst, welche den Stapel von Sensoren zu dem Abgabeende bewegen, und außerdem Sperrklinkenmittel innerhalb des Innengehäuses umfasst, welche eine Bewegung des Stapels von Sensoren in Richtung des gegenüberliegenden Endes verhindern oder inhibieren.

22. Kassette nach Anspruch 19 oder Anspruch 20, bei der das Innengehäuse der Kassette aus einem trocknenden Kunststoffmaterial gefertigt ist.

23. Kassette nach Anspruch 18, bei der das Dichtglied ein Ethylenpropylen-dien-terpolymer umfasst.

24. Kassette nach Anspruch 23, bei der das Dichtglied außerdem 0,2 bis 5 % eines antistatischen Erucamid-Mittels/Erucamid-Gleitmittels umfasst.

## Revendications

1. Cartouche (2) à utiliser dans un dispositif de distribution de détecteurs, ayant un boîtier extérieur (11) et une pluralité de détecteurs (6) disposés à l'intérieur les uns sur les autres en une pile, chaque détecteur (6) étant conçu pour tester une concentration d'une substance à analyser dans un fluide devant lui être appliqué ;
la cartouche ayant une première extrémité de distribution (13) et une deuxième extrémité opposée espacée d'une distance fixe, et la cartouche comprenant une première ouverture (15) pour l'éjection d'un détecteur situé au plus près de la première extrémité (13) et une deuxième ouverture (16) opposée à la première ouverture, pour l'accès au moyen d'un élément de poussée ;
dans laquelle la première ouverture (15) et la deuxième ouverture (16) sont chacune munies de moyens d'étanchéité (17) conformes qui sont au moins en partie disposés à l'extérieur du boîtier extérieur, chacun des moyens d'étanchéité (17) comprenant un tube en caoutchouc naturel ou synthétique ayant des première et deuxième surfaces d'étanchéité (17a, 17b) qui sont capables de coopérer pour former de manière non permanente un joint sensiblement étanche à l'humidité lorsque des forces de serrage appropriées leur sont appliquées.

2. Dispositif de distribution de détecteurs pour distribuer des détecteurs pour tester une concentration d'une substance à analyser dans un fluide devant lui être appliqué, le dispositif comprenant :
une cartouche selon la revendication 1,
un logement pour recevoir la cartouche ;
pour chacun desdits moyens d'étanchéité conformes, une paire d'éléments de serrage pour serrer de manière non permanente les moyens d'étanchéité pour former un joint sensiblement étanche à l'humidité ; et
un élément de poussée devant être inséré de manière réversible dans la deuxième ouverture quand les moyens d'étanchéité ne sont pas serrés, pour pousser le détecteur le plus près de la première extrémité par la première ouverture vers une position de distribution.

3. Dispositif selon la revendication 2, dans lequel chacun des moyens d'étanchéité est formé à partir d'un élastomère thermoplastique.

4. Dispositif selon la revendication 2 ou la revendication 3, dans lequel les moyens d'étanchéité sont co-moulés avec le boîtier extérieur de la cartouche.

5. Dispositif selon l'une quelconque des revendication 2 à 4, dans lequel le boîtier extérieur de la cartouche contient un ensemble interne comprenant un boîtier intérieur dans lequel se trouve une pile de détecteurs et qui a des ouvertures opposées qui coïncident avec les ouvertures correspondantes dans le boîtier extérieur, pour permettre l'entrée de l'élément de poussée et la sortie d'un détecteur.

6. Dispositif selon la revendication 5, comprenant en outre un moyen formant ressort dans le boîtier intérieur qui pousse la pile de détecteurs vers l'extrémité de distribution, et comprenant en outre un moyen formant cliquet dans le boîtier intérieur qui empêche ou interdit un mouvement de la pile de détecteurs vers l'extrémité opposée.

7. Dispositif selon la revendication 5 ou la revendication 6, dans lequel le boîtier intérieur de la cartouche est formé à partir d'une matière plastique déshydratante.

8. Dispositif selon l'une quelconque des revendication 2 à 7, comprenant en outre un châssis relié au logement et un mécanisme de sortie pour déployer l'élément de poussée depuis une position de repos à l'extérieur de la cartouche vers une position déployée dans laquelle il pousse un détecteur de la cartouche vers la position de distribution.

9. Dispositif selon la revendication 8, dans lequel le mécanisme de sortie comprend en outre un tambour pousseur monté de manière à tourner sur le châssis, et dans lequel l'élément de poussée est flexible et est au moins en partie enroulé autour du tambour pousseur lorsque les éléments d'étanchéité sont serrés.

10. Dispositif selon la revendication 8 ou la revendication 9, dans lequel l'actionnement du mécanisme de sortie pour déployer l'élément de poussée provoque le desserrage des moyens d'étanchéité avant que l'élément de poussée pénètre dans la deuxième ouverture et dans lequel le retrait de l'élément de poussée de la position déployée vers la position de repos provoque le serrage des moyens d'étanchéité après le retrait de l'élément de poussée de la deuxième ouverture.

11. Dispositif selon la revendication 10, dans lequel le mécanisme de sortie comprend en outre une came qui est reliée au tambour d'entraînement et peut tourner avec lui ; dans lequel la rotation initiale de la came oblige les éléments de serrage à desserrer les moyens d'étanchéité, et une rotation supplémentaire de la came oblige les éléments de serrage à serrer les moyens d'étanchéité.

12. Dispositif selon la revendication 11, dans lequel le mécanisme de sortie comprend un tambour d'entraînement et un ressort moteur reliant le tambour d'entraînement et le châssis ; le tambour d'entraînement étant monté de manière à tourner en liaison avec le tambour pousseur, et le tambour d'entraînement pouvant être tourné par un utilisateur depuis une position initiale afin d'armer le ressort moteur, et, dans lequel le mécanisme de sortie comprend en outre un moyen formant verrou pour raccorder de manière non permanente le tambour d'entraînement et le tambour pousseur lorsque le tambour d'entraînement a été tourné depuis sa position initiale vers une position prédéterminée d'accouplement de manière que le retour du tambour d'entraînement de sa position d'accouplement vers sa position initiale dû au déroulement du ressort moteur fasse tourner le tambour pousseur.

13. Dispositif selon la revendication 12, comprenant en outre un ressort de rappel reliant le tambour pousseur et le châssis, la rotation du tambour pousseur depuis une position initiale due au tambour d'entraînement enroulant le ressort de rappel de sorte que, lorsque le tambour pousseur est libéré du tambour d'entraînement, le ressort de rappel ramène le tambour pousseur dans sa position initiale.

14. Dispositif selon la revendication 9, dans lequel le mécanisme de sortie comprend en outre un premier pignon et un deuxième pignon et un élément d'entraînement qui est monté de manière rotative par rapport au châssis ; dans lequel lesdits pignons sont engrenés ensemble et l'un des pignons est fixé sur le tambour pousseur de sorte qu'ils tournent ensemble ; l'agencement étant tel que pendant une rotation complète de l'élément d'entraînement, il s'engrène avec le premier pignon et le fait tourner pendant une partie de sa course et s'engrène avec le deuxième pignon et le fait tourner pendant une deuxième partie de sa course, ainsi le tambour pousseur est tout d'abord entraîné de manière à provoquer l'extension de l'élément de poussée de la position de repos à la position déployée, puis est entraîné dans le sens inverse afin que l'élément de poussée revienne dans la position de repos.

15. Dispositif selon la revendication 9, dans lequel le mécanisme de sortie comprend en outre un pignon qui est fixé sur le tambour pousseur de sorte qu'ils tournent ensemble, et une crémaillère montée de manière à effectuer une translation réciproque par rapport au châssis ; l'agencement étant tel que pendant une translation de la crémaillère dans une première direction, elle ne s'engrène pas avec le pignon et que pendant une translation de la crémaillère dans une deuxième direction opposée, elle s'engrène avec le pignon et le fait tourner afin de faire tourner le tambour pousseur ; le dispositif comprenant en outre un ressort de rappel reliant le tambour pousseur et le châssis, la rotation du tambour pousseur depuis une position initiale enroulant le ressort de rappel de sorte que, lorsque le pignon est libéré de la crémaillère, le ressort de rappel ramène le tambour pousseur dans sa position initiale.

16. Dispositif selon l'une quelconque des revendication 2 à 15, dans lequel les détecteurs comprennent des biodétecteurs sur lesquels se trouve un moyen formant réactif pour produire un signal électrique en réponse à la concentration d'une substance à analyser dans un fluide appliqué, et des pistes d'électrode en contact avec le moyen formant réactif, et dans lequel le dispositif comprend en outre des contacts électriques montés en liaison avec le logement de façon à coopérer avec lesdites pistes d'électrode dans ladite position de distribution, et un appareil de mesure raccordé aux contacts comportant un moyen électronique pour produire une sortie de signal qui dépend du signal émis par un détecteur quand le détecteur coopère avec les contacts.

17. Dispositif pour mesurer une concentration d'une substance à analyser dans un fluide, comprenant un dispositif de distribution de détecteurs selon la revendication 2, un moyen formant réactif se trouvant sur chaque biodétecteur pour produire un signal électrique en réponse à la concentration d'une substance à analyser dans un fluide appliqué, et des pistes d'électrode en contact avec le moyen formant réactif;
le dispositif comprenant en outre :
des contacts électriques montés en liaison avec le logement de façon à coopérer avec lesdites pistes d'électrode dans ladite position de distribution, et un appareil de mesure raccordé aux contacts comportant un moyen électronique pour produire une sortie de signal qui dépend du signal émis par un détecteur quand le détecteur coopère avec les contacts.

18. Cartouche selon la revendication 1, dans laquelle chacun des moyens d'étanchéité est formé à partir d'un élastomère thermoplastique.

19. Cartouche selon l'une quelconque des revendication 1 ou 18, dans laquelle les moyens d'étanchéité sont moulés simultanément avec le boîtier extérieur de la cartouche.

20. Cartouche selon l'une quelconque des revendication 1, 18 ou 19, dans laquelle le boîtier extérieur de la cartouche contient un ensemble interne comprenant un boîtier intérieur dans lequel se trouve la pile de détecteurs et qui comporte des ouvertures opposées qui coïncident avec les ouvertures correspondantes dans le boîtier extérieur, pour permettre l'entrée de l'élément de poussée et la sortie d'un détecteur.

21. Cartouche selon la revendication 20, comprenant en outre un moyen formant ressort dans le boîtier intérieur qui pousse la pile de détecteurs vers l'extrémité de distribution, et comprenant en outre un moyen formant cliquet dans le boîtier intérieur qui empêche ou interdit un mouvement de la pile de détecteurs vers l'extrémité opposée.

22. Cartouche selon la revendication 19 ou la revendication 20, dans laquelle le boîtier intérieur de la cartouche est formé à partir d'une matière plastique déshydratante.

23. Cartouche selon la revendication 18, dans laquelle l'élément d'étanchéité comprend un terpolymère éthylène-propylène-diène.

24. Cartouche selon la revendication 23, dans laquelle l'élément d'étanchéité comprend en outre de 0,2 à 5% d'un agent antistatique / de glissance erucamide.
